# EUROPEAN PATENT APPLICATION

(11) **EP 3 858 436 A1**
(43) Date of publication of application: **04.08.2021**
(21) Application number: 21154065.3
(22) Date of filing: 28.01.2021
(51) Int. Cl.: A61N 5/10

(54) **AN AUTOMATED SYSTEM FOR PROVIDING QUALITY ASSURANCE OF RADIATION THERAPY WITH AN INTEGRATED RADIATION FIELD ANALYZE**

(30) Priority: 28.01.2020 IN 202041003695
(71) Applicant: Panacea Medical Technologies Pvt. Ltd, Karnataka 563130 (IN)
(72) Inventor: Subrahmanyam, Goteti Venkata, 563130 Karnataka (IN); Mani, Tamilarasan, 563130 Karnataka (IN); Hulmani, Girish, 563130 Karnataka (IN); Palanisamy, Manikandan, 563130 Karnataka (IN); Rao, Avinash Kuppa, 563130 Karnataka (IN); Kumar, Kamsala Manoj, 563130 Karnataka (IN)
(74) Representative: ABYOO

(57) **Abstract**

The present invention discloses an automated system for providing Quality assurance of radiation therapy with an integrated Radiation Field Analyzer (RFA), wherein the system (**100**) comprises a radiation therapy equipment (**101**) for emitting one or more radiation beams (**101a**) and an RFA (**102**) detachably integrated or permanently integrated to the radiation therapy equipment (**101**). Further, the integrated RFA (**102**) comprises one or more radiation detectors (**103**) for performing data dosimetry operations with assistance of 3 independent axis modules. Furthermore, the system (**100**) comprises a three-dimensional phantom (**104**) which is disposed on the couch tabletop (**101c**) or on a pedestal into the gantry ring during the process of performing data dosimetry operations on one or more radiation beams (**101a**).

## Description

### Technical field of the invention

The present invention discloses an automated system for providing quality assurance of radiation therapy with an integrated Radiation Field Analyzer (RFA). The invention particularly relates to a radiation therapy equipment with an integrated RFA for performing periodic quality assessments for radiation beams emitted by the radiation therapy equipment.

### Background of the invention

Medical equipment which are employed in patient safety are periodically tested to ensure optimum quality and performance. The biomedical industry deploys specialized testing equipment such as the Radiation Field Analyser (RFA) or 3-Dimensional water phantom which are stand-alone machines used for testing patient treatment delivery equipment. Currently, the treatment delivery equipment and quality assurance/testing equipment are independent entities and are required to be used sequentially which results in wastage of time and effort in configuring the equipment whenever it is required to be employed.

Furthermore, the cost of individually employing both, the treatment delivery equipment and quality assurance/testing equipment is extremely high. Additionally, it is a laborious and risky process to transfer data from the quality assurance/testing equipment to the treatment delivery equipment for the purpose of planning the appropriate course of treatment for a plurality of patients. Presently, there are no integrated solutions which provide quality assurance and treatment delivery in one enclosure to facilitate a faster and more accurate process.

The Patent Application No. JP2009520512A titled "Integrated quality assurance for radiation treatment implementation systems" discloses an integrated quality assurance technique for verifying alignment of radiation treatment delivery systems. In one embodiment, the radiation treatment source is commanded to move to a preset source position. An image of the radiation treatment source at its actual source position is generated and compared to a reference image to determine whether the radiation treatment source has achieved a preset source position. In one embodiment, the positioning system is instructed to move the target detector to a preset target position. An image of the target detector at its actual target location is generated and compared to the reference target image to determine if the positioning system has correctly achieved the preset target location.

The Patent No. GB2344985A titled "Flexible automated testing and checking of radiation devices" discloses a system and method for maintaining a network of multiple radiation devices such as linear accelerator radiation therapy devices. Automated specification testing and checking of the network of radiation therapy devices facilitates integrated analysis of collected information and system calibration. A network interface is provided for coupling the radiation devices to the network through dedicated personal computers (PCs). Databases of device history records and device specifications are maintained on a system calibration server for each of the multiple radiation devices. Dosimetry scanners operable with the radiation devices use a dedicated PC as a client user interface in communication with the server via the network and one radiation device. The radiation devices are responsive to the client user interface for undergoing an operation sequence with dosimetry scanner performing a series of tests.

Hence, there exists a need for a simple integrated solution for providing radiation therapy treatment and quality assurance of the radiation beams using a singular equipment.

### Summary of the invention:

The present invention overcomes the drawbacks of the prior art by disclosing an automated system for providing quality assurance of radiation therapy with an integrated Radiation Field Analyzer (RFA), wherein the system comprises a radiation therapy equipment for emitting one or more radiation beams and an RFA which is integrated with the radiation therapy equipment. The RFA comprises one or more radiation detectors for performing data dosimetry operations with the assistance of three independent axis modules, namely an axis 1 module, an axis 2 module and an axis 3 module, or by combination of circular, horizontal and vertical drives or by any means to provide first, second and third orthogonal axes in a three dimensional scanning of the radiation detector.

The RFA may be permanently integrated or detachably integrated to the radiation therapy equipment. In application where the RFA is permanently integrated in radiation therapy equipment, the RFA is retracted and accommodated within the gantry of the radiation therapy equipment after performing data dosimetry operations. The system comprises a three-dimensional phantom for accommodating a pre-defined measurement medium such as water, wherein the three-dimensional phantom may be disposed on a couch tabletop or on a pedestal into the gantry ring during the process of performing data dosimetry operations on one or more radiation beams.

The present invention provides an integrated solution for validating the performance of a radiation therapy equipment using an RFA which is in-built with the radiation therapy equipment that is primarily employed for providing radiation treatments to patients. The integrated solution reduces the overall cost and time which is dedicated towards employing two separate equipment for quality assurance and radiation therapy. Furthermore, the present invention is completely automated and controlled using a singular user interface thereby reducing the dependency on multiple manufacturers or suppliers.

### Brief description of the drawings:

The foregoing and other features of embodiments will become more apparent from the following detailed description of embodiments when read in conjunction with the accompanying drawings. In the drawings, like reference numerals refer to like elements.

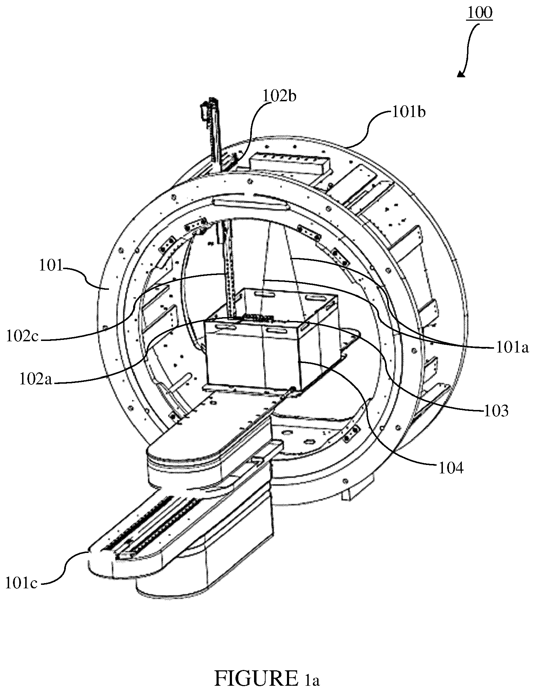

**FIG 1a** illustrates an automated system for providing quality assurance of radiation therapy with an integrated Radiation Field Analyzer (RFA) with a three-dimensional phantom disposed on a couch tabletop.

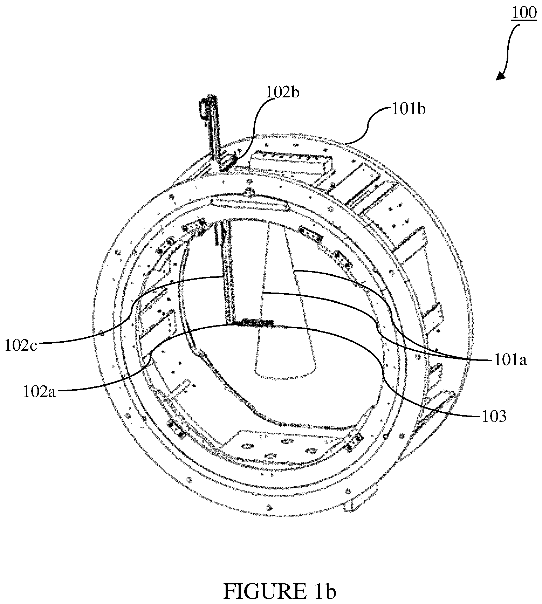

**FIG 1b** illustrates the gantry of a radiation therapy equipment which is integrated with a Radiation Field Analyzer (RFA).

### Detailed description of the invention:

Reference will now be made in detail to the description of the present subject matter, one or more examples of which are shown in figures. Each example is provided to explain the subject matter and not a limitation. Various changes and modifications obvious to one skilled in the art to which the invention pertains are deemed to be within the spirit, scope and contemplation of the invention.

The present invention discloses an automated system for providing Quality assurance of radiation therapy with an integrated Radiation Field Analyzer (RFA), wherein the system comprises a radiation therapy equipment for emitting one or more radiation beams and an RFA detachably integrated or permanently integrated to the radiation therapy equipment. Further, the integrated RFA comprises one or more radiation detectors for performing data dosimetry operations with assistance of three independent axis modules. Furthermore, the system comprises a three-dimensional phantom which may be disposed on the couch tabletop or on a pedestal into the gantry ring during the process of performing data dosimetry operations on one or more radiation beams.

**FIG 1a** illustrates an automated system for providing quality assurance of radiation therapy with an integrated Radiation Field Analyzer (RFA), wherein the system (**100**) comprises a radiation therapy equipment (**101**) for emitting one or more radiation beams (**101a**)**.** Further, the radiation therapy equipment (**101**) comprises a gantry (**101b**) which houses the source for emitting one or more radiation beams (**101a**) and a couch tabletop (**101c**) for enabling a linear motion of a patient within the inner periphery of the gantry **(101b).** Further, the system (**100**) comprises a Radiation Field Analyzer (RFA) (**102**) for performing periodic quality assurance assessments for one or more radiation beams (**101a**) emitted by the radiation therapy equipment (**101**).

In the present embodiment, the RFA (**102**) is integrated with the radiation therapy equipment (**101**), wherein the radiation therapy equipment (**101**) and RFA (**102**) comprise a singular user interface and control panel thereby eliminating the need for additional equipment for achieving synchronization between the radiation therapy equipment (**101**) and RFA (**102**). Further, parameters of the radiation therapy equipment (**101**) such as Beam ON & OFF controls, field size openings, collimator rotation, interlocks, and so on are controlled and monitored automatically in conjunction with RFA (**102**).

Further, in one embodiment, when RFA (**102**) is permanently integrated to the radiation therapy equipment (**101**), the RFA (**102**) is retractably extended to a pre-defined position for performing data dosimetry operation after which the RFA (**102**) is retracted and accommodated within the gantry (**101b**) of the radiation therapy equipment (**101**) such that the extension and retraction of the RFA (**102**) does not intervene during the process of radiation therapy provided by the radiation therapy equipment (**101**). In another embodiment, the RFA (**102**) may be detachably integrated to the radiation therapy equipment (**101**) and may be employed as per the requirement of one or more users.

Further, the RFA (**102**) comprises three-axis rectilinear modules namely, axis 1 module (**102a**), axis 2 module (**102b**) and axis 3 module (**102c**), wherein the operators for controlling the axis 1 module (**102a**), axis 2 module (**102b**) and axis 3 module (**102c**) are enclosed within the RFA (**102**) which provides the flexibility to be permanently integrated or detachably integrated with the radiation therapy equipment (**101**). In one embodiment, axis 1 module (**102a**) may house at least one radiation detector (**103**) for performing data dosimetry operations on one or more radiation beams (**101a**) which are emitted by the radiation therapy equipment (**101**). Further, the axis 2 module (**102b**) may allow the RFA (**102**) to move along the direction of y-axis and axis 3 module (**102c**) may allow the RFA (**102**) to move along the direction of z-axis.

In another embodiment, the axis 2 module (**102b**) may be secured to the structure of the gantry (**101b**) in the radiation therapy equipment (**101**), wherein the axis 1 module (**102a**) of the RFA (**102**) is mounted on the orthogonal axis i.e. axis 3 module (**102c**) of the RFA (**102**). However, the three axis modules may be interchanged and employed in multiple embodiments depending on the requirement of one or more users. Further, the system (**100**) comprises a three-dimensional phantom (**104**) for accommodating a pre-defined measurement medium such, water. The three-dimensional phantom (**104**) is disposed on the couch tabletop (**101c**) during the process of performing data dosimetry operations on one or more radiation beams (**101a**) which are emitted by the radiation therapy equipment (**101**)**.** In another embodiment, the three-dimensional phantom (**104**) may be disposed on a pedestal into the ring of the gantry (**101b**) during the process of performing data dosimetry operations on one or more radiation beams (**101a**).

**FIG 1b** illustrates the gantry of a radiation therapy equipment which is integrated with a Radiation Field Analyzer (RFA). The RFA (**102**) is integrated with the radiation therapy equipment (**101**) through the gantry **(101b).** Further, the integrated RFA (**102**) comprises one or more radiation detectors (**103**) for performing data dosimetry operations on one or more radiation beams **(101a)** with the assistance of three independent axis module namely axis 1 module **(102a),** axis 2 module **(102b)** and axis 3 module **(102c)** or by combination of circular, horizontal and vertical drives or by any means to provide first, second and third orthogonal axes in a three dimensional scanning of the radiation detector (**103**).

The present invention provides an integrated solution for validating the performance of a radiation therapy equipment (**101**) using an RFA (**102**) which is in-built with the radiation therapy equipment (**101**) that is primarily employed for providing radiation treatments to patients. The integrated solution reduces the overall cost and time which is dedicated towards employing two separate equipment for quality assurance and radiation therapy. Furthermore, the present invention is completely automated and controlled using a singular user interface thereby reducing the dependency on multiple manufacturers or suppliers.

While at least one exemplary embodiment has been presented in the foregoing detailed description, it should be appreciated that a vast number of variations exist.

**Reference numbers:**

| **Components** | **Reference Numbers** |
|---|---|
| System | **100** |
| Radiation therapy equipment | **101** |
| Radiation beam | **101a** |
| Gantry | **101b** |
| Couch tabletop | **101c** |
| Radiation Field Analyzer (RFA) | **102** |
| axis 1 module | **102a** |
| axis 2 module | **102b** |
| axis 3 module | **102c** |
| Radiation detector | **103** |
| Three-dimensional phantom | **104** |

## Claims

1. An automated system for providing quality assurance of radiation therapy with an integrated Radiation Field Analyzer (RFA), the system (**100**) comprising:
a. a radiation therapy equipment **(101)** for emitting one or more radiation beams **(101a),** wherein the radiation therapy equipment **(101)** comprises:
i. a gantry **(101b)** which houses the source for emitting one or more radiation beams **(101a);**
ii. a couch tabletop **(101c)** for enabling a linear motion of a patient within the inner periphery of the gantry (**101b**);
b. an RFA (**102**) which is integrated with the radiation therapy equipment (**101**) wherein the RFA (**102**) comprises an axis 1 module **(102a),** an axis 2 module **(102b)** and an axis 3 module **(102c)** for performing data dosimetry operations on one or more radiation beams **(101a);**
c. at least one radiation detector (**103**) for receiving and analyzing one or more radiation beams (**101a**) which are emitted by the radiation therapy equipment (**101**);
d. a three-dimensional phantom (**104**) for accommodating a pre-defined measurement medium, wherein the three-dimensional phantom (**104**) is disposed on the couch tabletop **(101c)** during the process of performing data dosimetry operations on one or more radiation beams **(101a).**

2. The system (**100**) as claimed in claim 1, wherein the radiation therapy equipment **(101)** and RFA **(102)** comprise a singular user interface and control panel.

3. The system (**100**) as claimed in claim 1, wherein the RFA (**102**) is detachably integrated or permanently integrated to the radiation therapy equipment **(101),**

4. The system (**100**) as claimed in claim 1, wherein the permanently integrated RFA (**102**) is retractably extended to a pre-defined position for performing data dosimetry operations, and retracted after performing data dosimetry operations and accommodated within the gantry **(101b)** of the radiation therapy equipment (**101**).

5. The system (**100**) as claimed in claim 1, wherein the RFA (**102**) which is integrated within the gantry **(101b)** of the radiation therapy equipment (**101**) does not intervene during the process of radiation therapy provided by the radiation therapy equipment (**101**).

6. The system (**100**) as claimed in claim 1, wherein the axis 1 module (**102a**) of the RFA (**102**) is mounted on the axis 3 module (**102c**) of the RFA (**102**).
